Europäisches Patentamt

European Patent Office

Office européen des brevets

(19)

(11) Numéro de publication: **0 133 391**
A1

## (12) DEMANDE DE BREVET EUROPEEN

(21) Numéro de dépôt: 84401448.0

(22) Date de dépôt: 06.07.84

(51) Int. Cl.⁴: **A 61 L 15/04**, A 61 L 15/06, A 61 K 47/00, A 61 K 49/00

(30) Priorité: 13.07.83 FR 8311716

(43) Date de publication de la demande: 20.02.85
Bulletin 85/8

(84) Etats contractants désignés: **AT BE CH DE FR GB IT LI LU NL SE**

(71) Demandeur: GUERBET S.A., 16-24 Rue Jean Chaptal, F-93601 Aulnay-sous-Bois Cedex (FR)

(72) Inventeur: **Trampont, Philippe**, 1 Rue de Béarn, F-92210 Saint-Cloud (FR)
Inventeur: **Madoule, Philippe**, 85 Avenue de Fécamp, F-75012 Paris (FR)
Inventeur: **Bonnemain, Bruno**, 9, Rue de Reims, F-77290 Mitry Mory (FR)
Inventeur: **Puisieux, Francis**, 66, Rue de Strasbourg, F-94700 Maisons-Alfort (FR)

(74) Mandataire: **Polus, Camille et al**, c/o Cabinet Lavoix 2, Place d'Estienne d'Orves, F-75441 Paris Cedex 09 (FR)

(54) Composition pour embolisation thérapeutique.

(57) La présente invention a pour objet une composition pour embolisation thérapeutique comprenant au moins un gel constitué par un agent gélifiant et un solvant pour cet agent gélifiant, caractérisée en ce qu'elle a une viscosité de 1 à 20 Pa.s mesurée à 37°C.

EP 0 133 391 A1

Composition pour embolisation thérapeutique
----------------

La présente invention concerne une composition pour embolisation thérapeutique.

L'embolisation thérapeutique est une technique qui consiste à amener dans un vaisseau sanguin une embole oblitérante.

Cette technique est utilisée à différentes fins thérapeutiques. Comme principales indications, on peut citer le contrôle des hémorragies, la dévascularisation des tumeurs, la réduction de la douleur dans le processus tumoral malin, le traitement des malformations vasculaires.

Différents matériaux d'embolisation ont été proposés. Comme exemples de tels matériaux, on peut citer des microbilles dures (verre, procelaine, acier, cire, matières plastiques), des éponges de gélatine, des substances fluides polymérisables, des substances dissoutes dans un solvant résorbable, des ballonnets détachables.

Certains de ces matériaux soit présentent une toxicité importante, soit sont d'une mise en oeuvre très délicate.

Afin de pouvoir suivre l'embolisation par des méthodes radiologiques, on utilise de préférence des matériaux radio-opaques, ou bien on utilise des matériaux non radio-opaques en mélange avec des produits de contraste.

C'est ainsi qu'on a proposé en particulier une composition lipophile comprenant un mélange d'une solution de zeïne dans de l'huile d'oeillette et de divers produits de contraste. ( D.NOVAK,J.WEBER,H.WICKERS,G.ZABEL. New liquid and semiliquid embolizing substances for tumour embolisation.Ann.Radiol.,1981,$\underline{24}$, 5,428-431.)

Par précipitation de la zeïne on forme une embole qui demeure de façon permanente dans le vaisseau oblitéré.

Par ailleurs, on a proposé d'utiliser des microsphères d'éponge de fer en suspension dans une solution aqueuse de polysaccharide (M. Sako et coll. Invest. Radiol. 1982, 17, 574). Cet article préconise d'utiliser des solutions dont la viscosité est inférieure à 200 centipoises (0,2 Pa.s) car au-delà la résistance à l'écoulement à travers les cathéters augmente trop fortement.

Or, contrairement à ce que l'on pouvait attendre, la Demanderesse a découvert qu'il était possible d'injecter dans les vaisseaux sanguins, par les moyens habituels, des compositions ayant des viscosités élevées et d'obtenir ainsi aisément des emboles.

Elle a découvert également que ces compositions constituaient des véhicules particulièrement appropriés pour diverses substances et notamment des substances solides, en particulier des produits opacifiants, des médicaments et des microsphères.

La présente invention a ainsi pour objet une composition pour embolisation thérapeutique comprenant au moins un gel constitué par un agent gélifiant et un solvant pour cet agent gélifiant, caractérisé en ce qu'elle a une viscosité de 1 à 20 Pa.s mesurée à 37°C.

Un grand nombre d'agents gélifiants peuvent être utilisés (seuls ou en mélange). Comme exemples, on peut citer notamment :

1. La cellulose et ses dérivés
   Cellulose
   Méthylcellulose
   Carboxyméthylcellulose sodique Divers éthers de la cellulose :
      Ethylcellulose
      Méthyléthylcellulose
      Hydroxy éthylcellulose
      Hydroxypropylméthylcellulose
      Hydroxyéthylméthylcellulose
   Acétophtalate de cellulose

2. Les gommes et polyosides divers

Amidons

Gomme arabique

Gomme adragante

Gomme de sterculia ou gomme karaya

Gomme guar ou guaranates

Pectines

Alginates

Carraghénates : lambda kappa

Gélose ou agar-agar

3. Les protéines

Gélatine

Caséine

4. Carbopols et dérivés (polymères de l'acide acrylique)

5. Acide silicique et dérivés

6. Les produits minéraux Silices diverses Silicates (kaolin, bentonites, silicates de magnésium et d'aluminium)

7. Le stéarate d'aluminium

Les dérivés cellulosiques se sont avérés particulièrement intéressants comme agent gélifiant dans la pratique de l'embolisation. Parmi ces dérivés, les plus intéressants sont les carboxyméthyl celluloses et les hydroxypropyl méthyl celluloses (présentant des groupes hydroxy non-substitués, ainsi que des groupes méthoxy et des groupes hydroxypropyl) et plus particulièrement les hydroxypropyl méthylcelluloses présentant un taux de groupes méthoxy représentant de 84 à 93 % des groupes substituants (Métoloses de type SH).

Le solvant pour l'agent gélifiant est choisi en fonction de l'agent gélifiant. Pour les agents gélifiants hydrophiles, on utilise un solvant hydrophile, notamment l'eau ou l'éthanol, ou des mélanges des deux, de préférence des mélanges eau/ éthanol contenant de 20 à 70 % en volume d'éthanol. L'incorporation d'éthanol est intéressante dans la mesure où l'éthanol peut jouer un rôle actif dans l'embolisation.

Pour les agents gélifants lipophiles tels que l'acide silicique, on utilise un solvant lipophile, tel que le DMI (1,4:3,6-dianhydro-2,5-di-O-méthyl D-glucitol ou diméthylisosorbide).

La composition peut également comprendre des mélanges de gels et, notamment, un mélange d'un gel hydrophile tel qu'un gel d'hydroxypropyl méthylcellulose dans l'eau, et d'un gel lipophile, tel qu'un gel d'acide silicique dans le DMI, ou encore d'un autre produit lipophile. Ceci permet d'incorporer dans la composition toute substance intéressante en adaptant le caractère hydrophile/lipophile de la composition à la substance à incorporer.

Les viscosités données ici sont celles mesurées à 37°C à l'aide d'un viscosimètre à mobile tournant.

Dans un mode de réalisation avantageux de la présente invention, le solvant contient à l'état dissous un produit opacifiant de type ionique ou de type non-ionique ou bien est lui-même constitué en totalité ou en partie par ce produit opacifiant.

Comme exemples de compositions pour embolisation contenant ainsi un agent opacifiant, on peut citer les gels hydrophiles comprenant comme solvant une solution aqueuse d'un sel d'un acide polyiodobenzénique tel que l'acide ioxitalamique ou l'acide ioxaglique et ayant une concentration de 20 à 70 g de sel pour 100 ml de gel, ainsi qu'un gel lipophile comprenant comme solvant des esters éthyliques des acides gras iodés de l'huile d'oeillette (Lipiodol ultra fluide) ou du monoiodostéarate d'éthyle (Duroliopaque).

Le solvant peut également contenir d'autres substances dissoutes et notamment des principes actifs, ainsi que divers adjuvants, en particulier des produits épaississants, des liants, des dispersants, des absorbants, des stabilisants. Comme exemple d'adjuvants, on peut citer les sucres, les Polyvidones, les polyéthylène glycols, les surfactifs (anioniques, cationiques et non-ioniques), ainsi que les sels de potassium.

La présente invention a également pour objet une composition pour embolisation thérapeutique du type précédemment décrit; qui comprend en outre une ou plusieurs substances solides.

Ces substances solides peuvent être notamment des particules de produits opacifiants,des particules de principes actifs,des microbilles ou microsphères,des microcapsules,des nanosphères, des nanocapsules,des liposomes ou leurs mélanges.

Ces substances peuvent être incorporés dans la composition généralement jusqu'au moins 25% en volume.

La taille des particules et substances analogues est seulement limitée par la méthode d'injection.C'est ainsi qu'on peut injecter des gels contenant des microsphères allant jusqu'à 800 microns de diamètre.

Pour les produits opacifiants,la taille moyenne des particules est avantageusement de 30 à 100 microns. Dans ce cas,on a constaté que la présence de telles particules de produits opacifiants facilitait l'injection du gel et diminuait l'incorporation de bulles d'air.On préfère,à cet effet,les produits opacifiants hydrophiles.

Pour les microbilles,la taille moyenne préférée est de 50 à 800 microns.

Il est à noter que les particules solides ainsi que des solutions de médicaments ou de produits de contraste peuvent être ajoutées au gel juste avant l'emploi, ce qui permet une grande liberté pour le praticien.

Les compositions selon l'invention peuvent être injectées par des moyens classiques tels qu'une seringue. L'administration peut se faire par les voies intra-artérielle,intra-veineuse,sous-cutanée,intra-musculaire ou par ponction directe.

Les doses à administrer sont fonction du territoire à emboliser.

Les compositions selon l'invention ont une durée d'embolisation qui varie avec la nature des agents gélifiants. Cette durée est plus élevée avec les agents gélifiants lipophiles qu'avec les agents gélifiants hydrophile.

6.  0133391

Dans le cas où il y a passage dans la circulation générale, il y a dilution immédiate du gel. Donc, le gel perd ses propriétés d'agent embolisant. Il n'y a donc pas de risque d'emboles secondaires à d'autres niveaux que celui recherché. C'est un facteur de sécurité supplémentaire.

Les exemples suivants illustrent l'invention.

EXEMPLE 1

On prépare des gels aqueux de carboxyméthyl cellulose sodique par dispersion sous agitation mécanique dans l'eau de différentes quantités de carboxyméthylcellulose sodique.

On mesure la viscosité des gels obtenus avec un viscosimètre à mobile tournant, modèle STV, vendu par la Société Contraves. Les valeurs de viscosité sont données dans le Tableau I suivant.

TABLEAU I

| Teneur en CMC (g/100ml) | Viscosité (Pa.s) à 37°C |
|---|---|
| 2 | 2 |
| 2,5 | 4 |
| 3 | 7 |
| 4 | 13 |
| 4,5 | 18 |

Tous les gels ainsi préparés sont administrables à l'aide d'une seringue et d'un cathéter d'un diamètre 5 French.

EXEMPLE 2

On prépare un gel en dispersant 2,5 % (Poids/ volume) de carboxyméthylcellulose sodique dans un mélange eau-éthanol contenant 40 % en volume d'éthanol.

La viscosité du gel est de 4 Pa.s (à 37°C).

Ce gel est injecté aisément en opérant comme à l'exemple 1.

Ce gel s'avère intéressant en raison du fait que, dans ces conditions, l'éthanol garde ses propriétés de précipitation des protéines sériques.

EXEMPLE 3

On prépare un gel comme à l'exemple 1 contenant 2,5 % (poids/volume) de carboxyéthylcellulose sodique dans de l'eau. On disperse dans le gel obtenu des particules d'acide ioxitalamique ayant une taille moyenne d'environ 50 microns. On peut incorporer ainsi jusqu'à 60 g d'acide ioxitalamique dans 100 ml de gel sans détruire la structure du gel. Des teneurs de 30 à 40 g d'acide ioxitalamique ont pour effet de faciliter l'injection du gel tout en diminuant l'incorporation des bulles.Au-delà de ces teneurs,l'effet sur l'injection n'est plus décelé.

Le gel est administré au niveau du territoire à emboliser au moyen d'une sonde pour artériographie et d'une seringue ordinaire.L'embolisation est suivie par contrôle radiologique.

EXEMPLE 4

En opérant comme à l'exemple 1 on obtient des gels pour embolisation ayant les caractéristiques indiquées dans le Tableau II.Ces gels sont testés comme décrit aux exemples 1 et 3.

TABLEAU II

| Agent gélifiant | % en poids | Solvant | Viscosité Pa.s à 37°C | Facilité d'injection | Possibilité d'incorporation d'un produit opacifiant en poudre - |
|---|---|---|---|---|---|
| Méthylcellulose | 2 | eau | 3 Pa.s | ++ | +++ |
| Carraghénate (Lambda) | 8 | eau | 13 Pa.s | +++ | +++ |
| Carbopol 934 neutralisé avec NaOH | 0,5 | eau | 3 Pa.s | ++ | + |
| Acide silicique | 10 | Lipiodol | 2 Pa.s | +++ | ++ |

EXEMPLE 5

On prépare un gel contenant 10% en poids d'acide silicique dans du Lipiodol ultra fluide (esters éthyliques des acides gras iodés de l'huile d'oeillette).

On obtient un gel aisément injectable et ayant de bonnes propriétés opacifiantes.

EXEMPLE 6

On prépare un gel contenant 2,5% de carboxyméthylcellulose sodique dans de l'eau. On disperse dans le gel 20% (vol/vol) de microsphères de cire de carnauba, d'acide polylactique ou de sérum-albumine ayant une taille moyenne de 300 microns.

On obtient un gel qui est aisément injectable.

EXEMPLE 7

On prépare un gel comme à l'exemple 1 contenant 2,5% en poids de carboxyméthylcellulose sodique. On mélange 100 parties en volume de ce gel avec 30 parties en volume de Télébrix (solution aqueuse de ioxitalamate de sodium et de méglumine à 38% d'iode). On obtient un gel aisément injectable.

EXEMPLE 8

On prépare un gel en dispersant 2% en poids de carboxyméthylcellulose sodique dans une solution de Télébrix diluée de moitié avec de l'eau. On obtient également un gel injectable.

EXEMPLE 9

On prépare un gel comme à l'exemple 1 en dispersant 3,6 % (poids/volume) d'hydroxypropyl méthylcellulose de type Metolose 60 SH 4000 vendu par la Société Seppic.

On stérilise le gel obtenu à 110°C pendant 20 mn. Après stérilisation, le gel a une viscosité de 4 Pa.s (mesurée à 37°C). Ce gel est aisément injectable. Il est stable à 37°C.

EXEMPLE 10

On opère comme à l'exemple 9 en dispersant 2,5 % (poids/volume) de la même hydroxypropyl methyl Cellulose dans un mélange eau/éthanol 1/2).

On obtient un gel ayant une viscosité de 2,3 Pa.s (mesurée à 37°C). Ce gel est aisément injectable.

EXEMPLE 11

On prépare un gel comme à l'exemple 9 en dispersant 3,5 % (poids/volume) de la même hydroxypropyl méthylcellulose dans de l'Hexabrix (solution aqueuse de sel de sodium et de méthylglucamine de l'acide ioxaglique à 32 % d'iode).

On obtient un gel ayant une viscosité de 14,5 Pa.s (mesurée à 37°C). Ce gel est aisément injectable.

EXEMPLE 12

On prépare une composition sous forme de gel par mélange par parties égales d'un gel obtenu par dispersion de 3,5 % (poids/volume) de la même hydroxypropyl méthylcellulose qu'à l'exemple 9 et d'un gel obtenu par dispersion de 10 % d'acide silicique (poids/volume) dans du DMI.

Le mélange est un gel homogène et stable à 37°C.

Ce gel a une viscosité d'environ 4 Pa.s (mesurée à 37°C) et est aisément injectable.

EXEMPLE 13

On prépare un gel comme à l'exemple 1 contenant 3,6 % (poids/volume) d'hydroxypropyl méthylcellulose. On mélange une partie de ce gel avec une partie d'alcool absolu et une partie de Lipiodol ultrafluide.

On obtient un gel injectable ayant une viscosité d'environ 5 Pa.s (mesurée à 37°C).

REVENDICATIONS

1. Composition pour embolisation thérapeutique comprenant au moins un gel constitué par un agent gélifiant et un solvant pour cet agent gélifiant, caractérisée en ce qu'il a une viscosité de 1 à 20 Pa.s mesurée à 37°C.

2. Composition selon la revendication 1, caractérisée en ce que l'agent gélifiant est un dérivé cellulosique.

3. Composition selon la revendication 2, caractérisée en ce que l'agent gélifiant est une carboxyméthyl cellulose ou une hydroxypropyl méthylcellulose.

4. Composition selon l'une quelconque des revendications 1 à 3, caractérisée en ce que le solvant est choisi parmi l'eau, l'éthanol et leur mélange.

5. Composition selon la revendication 4, caractérisée en ce que le solvant est un mélange eau/éthanol contenant 20 à 70 % en volume d'éthanol.

6. Composition selon l'une quelconque des revendications 1 à 5, caractérisée en ce que le solvant contient un produit opacifiant.

7. Composition selon l'une quelconque des revendications 1 à 5, caractérisée en ce que le solvant est un produit opacifiant.

8. Composition selon l'une quelconque des revendications 1 à 7, caractérisée en ce qu'elle comprend en outre une ou plusieurs substances solides.

9. Composition selon la revendication 8, caractérisée en ce que les substances solides sont choisies parmi des particules de produits opacifiants, des particules de principes actifs, des microsphères, des microcapsules, des nanosphères, des nanocapsules, des liposomes ou leurs mélanges.

10. Composition selon la revendication 8 ou la revendication 9, caractérisée en ce que la ou les substances solides représentent jusqu'à 25 % en volume.

11. Composition selon l'une quelconque des revendications 8 à 10, caractérisée en ce qu'elle comprend des particules solides de produits opacifiants ayant une taille moyenne de 30 à 100 microns.

12. Composition selon l'une quelconque des revendications 8 à 11, caractérisée en ce qu'elle comprend des microbilles ayant une taille moyenne de 50 à 800 microns.

13. Composition selon la revendication 1, caractérisée en ce qu'elle comprend un mélange d'un gel hydrophile et d'un gel ou d'un autre produit lipophile.

1. Procédé de préparation d'une composition pour embolisation thérapeutique comprenant au moins un gel constitué par un agent gélifiant et un solvant pour cet agent gélifiant, caractérisé en ce que l'on règle la viscosité du gel à une valeur de 1 à 20 Pa.s mesurée à 37°C.

2. Procédé selon la revendication 1, caractérisé en ce que l'agent gélifiant est un dérivé cellulosique.

3. Procédé selon la revendicqtion 2, caractérisé en ce que l'agent gélifiant est une carboxyméthyl cellulose ou un hydroxypropyl méthylcellulose.

4. Procédé selon l'une quelconque des revendications 1 à 3, caractérisé en ce que le solvant est choisi parmi l'eau, l'éthanol et leur mélange.

5. Procédé selon la revendication 4, caractérisé en ce que le solvant est uh mélange eau/éthanol contenant 20 à 70% en volume d'éthanol.

6. Procédé selon l'une quelconque des revendications 1 à 5, caractérisé en ce que le solvant contient un produit opacifiant.

7. Procédé selon l'une quelconque des revendications 1 à 5, caractérisé en ce que le solvant est un produit opacifiant.

8. Procédé selon l'une quelconque des revendications 1 à 7, caractérisé en ce que l'on ajoute en outre une ou plusieurs substances solides.

9. Procédé selon la revendication 8, caractérisé en ce que les substances solides sont choisies parmi des particules de produits opacifiants, des particules de principes actifs, des microsphères, des microcapsules, des nanosphères, des nanocapsules, des liposomes ou leurs mélanges.

10. Procédé selon la revendication 8 ou la revendication 9, caractérisé en ce que la ou les substances solides représentent jusqu'à 25% en volume.

11. Procédé selon l'une quelconque des revendications 8 à 10, caractérisé en ce que l'on ajoute des particules

...

solides de produits opacifiant ayant une taille moyenne de 30 à 100 microns.

12. Procédé selon l'une quelconque des revendications 8 à 11, caractérisé en ce que l'on ajoute des microbilles ayant une taille moyenne de 50 à 800 microns.

13. Procédé selon la revendication 1, caractérisé en ce que l'on mélange un gel hydrophile et un gel ou un autre produit lipophile.

0133391

Numéro de la demande

**Office européen des brevets**

**RAPPORT DE RECHERCHE EUROPEENNE**

EP  84  40  1448

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | BIOLOGICAL ABSTRACTS, vol. 66, 1978, réf. no. 43504; H.A. MITTY et al.: "Partial combolization of large peripheral hemangioms for pain control" & RADIOLOGY 127(3), 671-672, 1978 * Abrégé * | 1,6 | A 61 L   15/04<br>A 61 L   15/06<br>A 61 K   47/00<br>A 61 K   49/00 |
| | --- | | |
| Y | FR-A-1 593 518   (PARACHEM CORP.) * Page 4, résumé * | 1-3 | |
| | --- | | |
| Y | FR-A-2 273 555   (PHARMACIA AKTIEBOLAG) * Pages 33-36 * | 1,2,6-12 | |
| | --- | | |
| Y | FR-A-2 273 516   (PHARMACIA AKTIEBOLAG) * Pages 30-32 * | 1,2,6-12 | DOMAINES TECHNIQUES RECHERCHES (Int. Cl.4) |
| | --- | | |
| A | CHEMICAL ABSTRACTS, vol. 97, no. 5, 2 août 1982, page 44, no. 33361z, Columbus, Ohio, USA; H. DAHLKE et al.: "Experimental pancreatic duct obstruction with an alcoholic prolamine solution producing a pancreatic execrine etrophy" & ADV. BIOMATER. 1982, 3, 759-765 | | A 61 L<br>A 61 K |
| | --- | | |
| A | GB-A-  700 150   (OTTO BRAUN) | | |
| | ---                        -/- | | |

Le présent rapport de recherche a été établi pour toutes les revendications

| Lieu de la recherche | Date d'achèvement de la recherche | Examinateur |
|---|---|---|
| LA HAYE | 12-10-1984 | REMPP G.L.E. |

## Office européen des brevets

## RAPPORT DE RECHERCHE EUROPEENNE

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 044 624 (E.R. SQUIBB & SONS INC.) ----- | | |

DOMAINES TECHNIQUES RECHERCHES (Int. Cl.4)

Le présent rapport de recherche a été établi pour toutes les revendications

| Lieu de la recherche LA HAYE | Date d'achèvement de la recherche 12-10-1984 | Examinateur REMPP G.L.E. |
|---|---|---|